# EUROPEAN PATENT SPECIFICATION

(11) **EP 4 184 157 B1**
(45) Date of publication and mention of the grant of the patent: **07.05.2025**
(21) Application number: 20944988.3
(22) Date of filing: 17.07.2020
(51) Int. Cl.: G01N 27/62, H01J 49/00, G16C 20/00

(54) **MASS SPECTROMETRY METHOD**
MASSENSPEKTROMETRIEVERFAHREN
PROCÉDÉ DE SPECTROMÉTRIE DE MASSE

(43) Date of publication of application: 24.05.2023
(73) Proprietor: HITACHI HIGH-TECH CORPORATION, Tokyo 105-6409 (JP)
(72) Inventor: HIROSE, Ryusuke, Tokyo 105-6409 (JP); AKIYAMA, Hideyuki, Tokyo 105-6409 (JP); SAKAI, Noriaki, Tokyo 105-6411 (JP)
(74) Representative: MERH-IP Matias Erny Reichl Hoffmann Patentanwälte PartG mbB
(86) International application number: PCT/JP2020/027826
(87) International publication number: WO 2022/014037

(56) References cited:
- WO-A1-2018/138901
- WO-A1-2018/138901
- JP-A- 2006 322 899
- JP-A- 2010 066 036
- JP-A- 2010 066 036
- JP-A- 2013 231 715
- JP-A- 2013 231 715
- JP-B2- 4 275 864
- US-A1- 2006 255 259
- US-A1- 2015 340 216

## Description

### Technical Field

The present disclosure relates to a mass spectrometry method and a mass spectrometer.

### Background Art

When a measurement target is analyzed by a mass spectrometer, it is necessary to determine whether a peak of an obtained mass spectrum is derived from a component to be analyzed or from a factor other than the component to be analyzed. In particular, when isotopes or homologs exist in the component to be analyzed, the number of peaks of the mass spectrum increases. For example, a large number of components having different numbers of carbon atoms and chlorine atoms exist in a chlorinated paraffin, and the number of peaks may reach several hundred in a mass spectrum obtained by measurement.

In general, an isotope abundance ratio is known, and a result reflecting the isotope abundance ratio is obtained also in the mass spectrum. PTL 1 discloses a technique for performing qualitative analysis of a sample on the basis of an isotope abundance ratio of a component to be analyzed.

PTL 2 describes a method which derives a monoisotopic ion peak from a compound whose element composition is unknown. The mass spectrometric data processing method includes the following steps: As an estimation model an approximate expression is used, which is based on the result obtained by analyzing a relationship between mass and the number of constituent elements of known compounds belonging to the same category as a target compound. A chemical formula is estimated on the basis of the estimation model when the measured mass corresponding to a peak is given. The isotope distribution on a mass spectrum is discriminated by fitting using a theoretical isotope distribution, in an attempt to identify a monoisotopic ion Further examples of known mass spectrometry methods also are disclosed in PTL3 and PTL4, respectively.

### Citation List

### Patent Literature

PTL 1: JP-A-2010-66036
PTL 2: JP 2013 231715
PTL 3: US2006-255259 A1
PTL 4: WO2018-138901 A1

### Summary of Invention

### Technical Problem

However, when there is a peak caused by a factor other than the component to be analyzed, since the peak is also analyzed, an erroneous determination is caused in qualitative analysis.

Therefore, the disclosure provides a technique for easily avoiding an influence of a component other than the component to be analyzed in mass spectrometry.

### Solution to Problem

The above stated problem is solved by a mass spectrometry method according to claim 1.

Preferred embodiments of the invention are defined in the dependent claims.

Further features related to the disclosure will become apparent from a description of the present specification and the accompanying drawings. In addition, aspects of the disclosure may be achieved and implemented using elements, combinations of various elements, the following detailed description, and accompanying claims.

The description of the present specification is merely exemplary, and is not intended to limit the scope of claims or application examples of the disclosure in any sense. Advantageous Effects of Invention

According to the technique of the disclosure, an influence other than the component to be analyzed can be easily avoided.

Problems, configurations, and effects other than those described above will become apparent from the following description of the embodiments and exemplary configurations.

### Brief Description of Drawings

[FIG. 1] FIG. 1 is a functional configuration diagram of a mass spectrometer according to a first exemplary configuration.
[FIG. 2] FIG. 2 is a flowchart of a mass spectrometry method according to the first exemplary configuration.
[FIG. 3] FIG. 3 is a diagram illustrating an example of a theoretical mass spectrum.
[FIG. 4] FIG. 4 is a diagram illustrating an example of a peak of a mass spectrum obtained by measurement.
[FIG. 5] FIG. 5 is a flowchart of a mass spectrometry method according to a first embodiment.
[FIG. 6] FIG. 6 is a flowchart of a mass spectrometry method according to a second embodiment.
[FIG. 7] FIG. 7 is a conceptual diagram of a method for separating an ion intensity.
[FIG. 8] FIG. 8 is a flowchart of a mass spectrometry method according to a third embodiment.

### Description of Embodiments and exemplary configurations

### [First exemplary configuration]

### <Configuration Example of Mass Spectrometer>

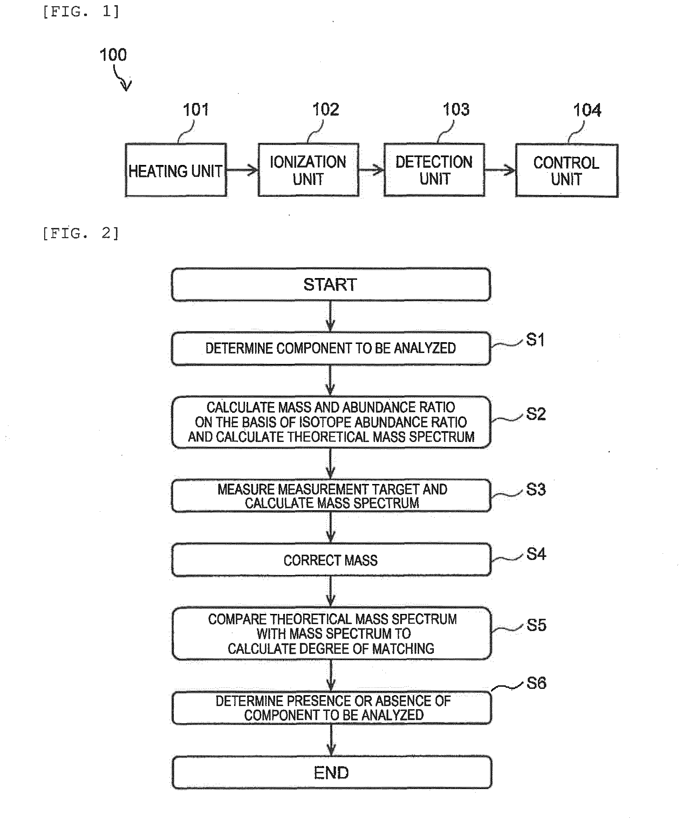

FIG. 1 is a functional configuration diagram of a mass spectrometer 100 according to a first exemplary configuration. In the present configuration, a case in which the mass spectrometer 100 is a thermal desorption mass spectrometer will be described as an example. The thermal desorption mass spectrometer heats a measurement target (sample) to generate a gas component, ionizes the gas component, and performs mass spectrometry on the gas component. A mass spectrometer to which the technique of the disclosure can be applied is not limited to the thermal desorption mass spectrometer, and the technique of the disclosure can be applied to a gas chromatograph mass spectrometer or a liquid chromatograph mass spectrometer that separates a compound in a measurement target by a separation column.

As illustrated in FIG. 1, the mass spectrometer 100 includes a heating unit 101 (preprocessing unit), an ionization unit 102 (preprocessing unit), a detection unit 103 (mass detection unit), and a control unit 104 (calculation unit).

The heating unit 101 heats a measurement target to generate a gas component. The heating unit 101 can include, for example, a heating furnace, and the measurement target can be conveyed by an autosampler to a heating chamber of the heating furnace.

The ionization unit 102 can include a known ionization device, and ionizes the gas component generated by the heating unit 101. Examples of an ionization method of the ionization unit 102 include atmospheric pressure chemical ionization (APCI) method, electrospray ionization (ESI) method, atmospheric pressure photoionization (APPI) method, and electron ionization (EI) method. Among them, atmospheric pressure chemical ionization method is less likely to cause destruction of a structure of a component to be analyzed in ionization (fragmentation of gas components) and is less likely to cause a fragment peak, and thus can detect the component to be analyzed without separation by a chromatograph or the like.

The detection unit 103 can include a known mass analyzer, and detects a mass of ions ionized by the ionization unit 102 and the number of ions (ion intensities) for each mass. The detection unit 103 outputs a detection signal of the ion intensities to the control unit 104. In addition, the detection unit 103 may output an ion current to the control unit 104 as a detection signal.

The control unit 104 calculates a mass spectrum on the basis of the mass of the ions and the number of ions for each mass detected by the detection unit 103, and analyzes the measurement target. In addition, the control unit 104 controls the overall operation of the mass spectrometer 100. The control unit 104 can include, for example, a memory in which a program for operating each unit of the mass spectrometer 100 is stored and a processor (CPU, MPU, or the like) that executes the program. The control unit 104 can be incorporated into a computer terminal such as a personal computer or a smartphone, and the control unit 104 is connected to, a storage device that stores various data, an input device for a user to input an instruction to the mass spectrometer 100, a display device that displays a mass spectrometry result or various GUI screens, or the like.

### <Mass Spectrometry Method>

FIG. 2 is a flowchart illustrating a mass spectrometry method according to the first exemplary configuration.

### (Step S1)

The user of the mass spectrometer 100 determines a component to be analyzed. Specifically, the control unit 104 displays a GUI screen for the user to determine the component to be analyzed on the display device, and the user uses the input device to input a desired component to be analyzed via the GUI screen. Here, data on a compound that can be the component to be analyzed may be stored as a database, or the user may select a compound from the database. In addition, the user may input a chemical formula (molecular formula, structural formula, or the like) of the component to be analyzed. Information of the input component to be analyzed is output to the control unit 104. In the present configuration, a case in which the component to be analyzed is a chlorinated paraffin will be described as an example.

### (Step S2)

The control unit 104 calculates, on the basis of the molecular formula of the component to be analyzed and an isotope abundance ratio, a mass of each isotope and an abundance ratio, and calculates a theoretical mass spectrum. The molecular formula of the compound that can be the component to be analyzed and the isotope abundance ratio may be stored as a database and read by the control unit 104, or may be calculated by the control unit 104 according to the chemical formula of the component to be analyzed input by the user.

The chlorinated paraffin is a generic term for a compound in which chlorine is bonded to an alkane (molecular formula: CₙH₂ₙ₊₁), and is a mixture of components having different numbers of carbon atoms and chlorine atoms. Therefore, a molecular formula of the chlorinated paraffin is represented by CₓH_{2x+2-y}Cl_{y}, where x represents the number of carbon atoms and y represents the number of chlorine atoms. Chlorine has two stable isotopes, i.e., ³⁵Cl and ³⁷Cl, and isotope abundance ratios thereof are ³⁵Cl (75.77%) and ³⁷Cl (24.33%). Therefore, even if x and y are the same, y + 1 types of isotopes having different masses exist.

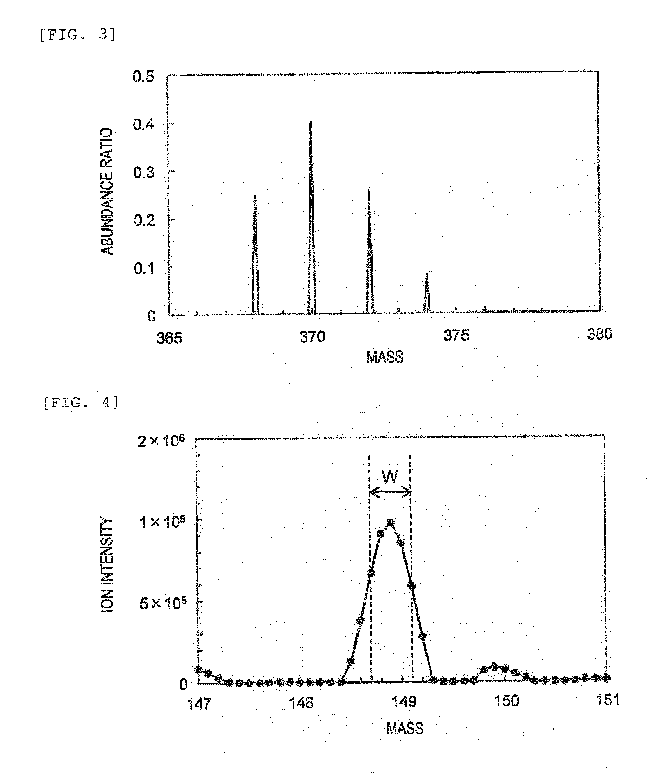

FIG. 3 is a theoretical mass spectrum calculated from the mass and the abundance ratio calculated for x = 14 and y = 5. A horizontal axis represents the mass, a vertical axis represents the abundance ratio calculated on the basis of the isotope abundance ratio of chlorine, and a total value of six peaks is calculated to be 1. The theoretical mass spectrum can be calculated by performing the same calculation on all combinations of x and y to be analyzed.

### (Step S3)

Returning to FIG. 2, the control unit 104 measures a measurement target suspected of including the component to be analyzed by the mass spectrometer 100, and acquires a mass spectrum (hereinafter, may be referred to as a "first mass spectrum"). Specifically, the control unit 104 drives the heating unit 101 and the ionization unit 102 to gasify and ionize the component to be analyzed, and receives an input of the detection signal of the detection unit 103. The control unit 104 obtains the mass spectrum with the mass (mass-to-charge ratio m/z) as a horizontal axis and the ion intensity as a vertical axis.

### (Step S4)

The control unit 104 corrects a mass of the mass spectrum acquired in step S3 in accordance with an ionization reaction in the ionization unit 102. For example, when oxygen ions (O₂⁻) are added in the ionization, the control unit 104 shifts the horizontal axis of the obtained mass spectrum to a minus side by 32Da. That is, the control unit 104 moves a peak at 448Da on the horizontal axis to 416Da.

### (Step S5)

The control unit 104 compares the theoretical mass spectrum calculated in step S2 with the mass spectrum acquired in step S3 to calculate a degree of matching. In the calculation of the degree of matching, a peak that is a comparison target is only a mass in which a peak of the theoretical mass spectrum exists, and the other peaks are not the comparison target. Accordingly, even if there is a peak other than the component to be analyzed in the mass spectrum acquired in step S3, the peak can be excluded from the comparison target. Here, the peak other than the component to be analyzed is caused by the mass spectrometer 100 itself, a container of the measurement target, impurity components other than the component to be analyzed included in the measurement target, and the like.

There are y + 1 types of peaks in the theoretical mass spectrum of the chlorinated paraffin, among which the peak that is the comparison target is not limited. For example, all peaks may be the comparison target, a plurality of peaks having strong intensities among peaks of the mass spectrum acquired in step S3 may be the comparison target, or peaks exceeding a predetermined threshold may be the comparison target.

A method of calculating the degree of matching is not particularly limited, and, for example, a correlation coefficient can be used.

FIG. 4 is a diagram illustrating an example of a peak of a mass spectrum obtained by mass spectrometry. As illustrated in FIG. 4, in general, each peak of the mass spectrum obtained by the mass spectrometer is not a single line but spreads to some extent, for example, spreads with a Gaussian function. Depending on the measurement, the spread may change with time, or peak positions may shift.

This makes a calculated value of the degree of matching unstable. Therefore, the control unit 104 can stabilize the value of the degree of matching by calculating, with respect to the peak of the mass spectrum obtained in step S3, an average value of the ion intensities within a range of a predetermined width W on the horizontal axis and using the average value for the calculation of the degree of matching.

### (Step S6)

Returning to FIG. 2, the control unit 104 determines presence or absence of the component to be analyzed on the basis of the degree of matching. The control unit 104 determines that the component to be analyzed is "present" when the degree of matching is equal to or larger than a threshold set in advance, and determine that the component to be analyzed is "absent" when the degree of matching is smaller than the threshold. In accordance with the invention, the control unit 104 sets a first threshold and a second threshold larger than the first threshold in advance, determine that the component to be analyzed is "absent" when the degree of matching is equal to or smaller than the first threshold, and determine that the component to be analyzed is "present" when the degree of matching is equal to or larger than the second threshold.

### <Summary of First Exemplary Configuration>

As described above, in accordance with the invention, the degree of matching between the mass spectrum obtained by the measurement and the theoretical mass spectrum is calculated only for the mass in which the peak of the theoretical mass spectrum of the component to be analyzed exists, and the presence or absence of the component to be analyzed is determined on the basis of the degree of matching. Accordingly, in the mass spectrum obtained by the measurement, even if there is a peak in a mass other than the component to be analyzed, the peak is not used for the calculation of the degree of matching. Therefore, since an influence of the peak other than the component to be analyzed can be avoided, an erroneous determination in qualitative analysis can be prevented.

The mass spectrometry method according to the present configuration is not limited to the above analysis of the chlorinated paraffin, and can be applied to analysis of a compound containing an element in which a plurality of stable isotopes exist and the isotope abundance ratio is not negligible. In the mass spectrometry method according to the present configuration, for example, an organic compound such as an organic halogen compound or an organic metal compound may be used as the component to be analyzed. As the organic halogen compound, organic chlorine compounds such as chlorinated paraffins or dioxins, and organic bromine compounds such as bromine-based flame retardants (for example, tetrabromobisphenol A) or brominated dioxins can be used as the component to be analyzed.

### [First Embodiment]

Above a method of calculating the degree of matching by calculating the theoretical mass spectrum of the component to be analyzed and the mass spectrum obtained by measuring the measurement target is described. The mass spectrum obtained by measuring the measurement target includes not only the compound contained in the measurement target, but also the peak caused by an element other than the measurement target such as the mass spectrometer 100 itself or the container of the measurement target. When the element other than the measurement target has the same mass as the component to be analyzed, that is, when a peak of the element other than the measurement target is a peak at the same position as the component to be analyzed, an adverse influence (erroneous determination) occurs in the measurement. Therefore, in the first embodiment, a technique for reducing an influence of the element other than the measurement target is proposed.

A mass spectrometer according to the present embodiment can be the same as the mass spectrometer 100 described in the first exemplary configuration.

### <Mass Spectrometry Method>

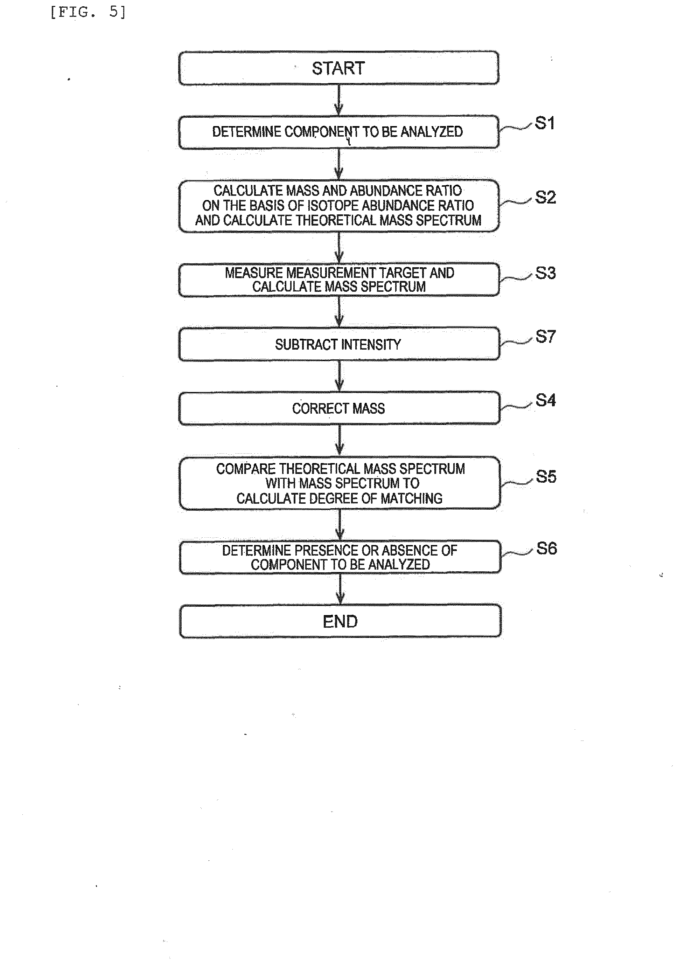

FIG. 5 is a flowchart of a mass spectrometry method according to the first embodiment. Steps S1 to S6 are the same as those in the first exemplary configuration, and thus the description thereof will be omitted. In the present embodiment, step S7 is performed before step S5. In step S7, the control unit 104 subtracts a mass spectrum (hereinafter, may be referred to as a "second mass spectrum") measured in advance by operating the mass spectrometer 100 in a state where the measurement target is absent from the mass spectrum (first mass spectrum) obtained in step S3. Specifically, the control unit 104 subtracts an ion intensity of a peak of the second mass spectrum from the ion intensity of the peak of the first mass spectrum. Here, as described with reference to FIG. 3, the calculation of this step can be performed with the average value of the ion intensities at the predetermined width W of each peak as the ion intensity of the peak. The second mass spectrum includes only the peak caused by the element other than the measurement target such as the mass spectrometer 100 or the container of the measurement target. Therefore, the mass spectrum of only the measurement target can be obtained by the processing of step S7. By comparing the mass spectrum with the theoretical mass spectrum, erroneous determination can be prevented.

Step S7 is only required to be performed between step S3 and step S5, and an order of step S4 and step S7 is not limited. In addition, the acquisition of the mass spectrum in the state where the measurement target is absent can be performed before measuring the measurement target in step S3.

### <Summary of First Embodiment>

As described above, in the first embodiment, the second mass spectrum acquired in the state where the measurement target is absent is subtracted from the first mass spectrum obtained by measuring the measurement target, and the degree of matching between the mass spectrum obtained by the subtraction and the theoretical mass spectrum of the component to be analyzed is calculated. Accordingly, since the influence of the peak caused by the elements other than the measurement target can be eliminated, analysis accuracy can be further improved as compared with the first exemplary configuration.

### [Second Embodiment]

In the first embodiment and first exemplary configuration, a technique for analyzing in consideration of the isotope abundance ratio of the element in the component to be analyzed is described. Depending on the component to be analyzed, peak positions may overlap among homologs, which may lead to erroneous determination if peak intensities of the homologs are not separated from each other. Therefore, in the second embodiment, a technique for separating the peaks overlapping among the homologs is proposed.

A mass spectrometer according to the present embodiment can be the same as the mass spectrometer 100 described in the first exemplary configuration.

### <Mass Spectrometry Method>

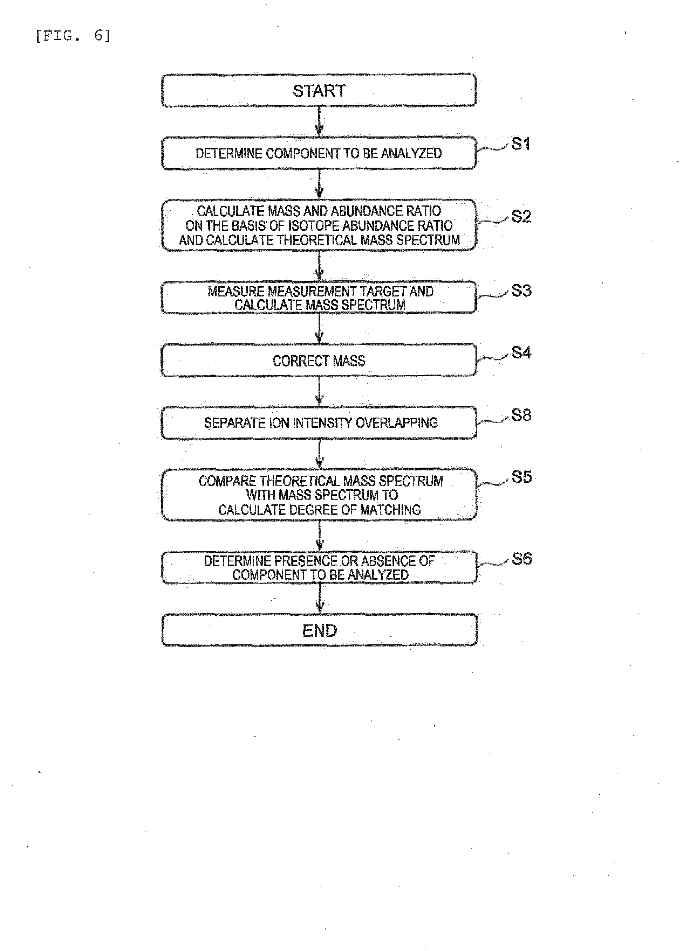

FIG. 6 is a flowchart of a mass spectrometry method according to the second embodiment. Steps S1 to S6 are the same as those in the first exemplary configuration, and thus the description thereof will be omitted. In the present embodiment, step S8 is performed before step S5. In step S8, the control unit 104 separates the ion intensities of the peaks of the mass spectrum obtained in step S3 when there is a peak at a position having the same mass between components (homologs) in which combinations of the number of carbon atoms x and the number of chlorine atoms y are different in the theoretical mass spectrum. An example of a method for separating the ion intensity (overlapping of peaks) will be described below.

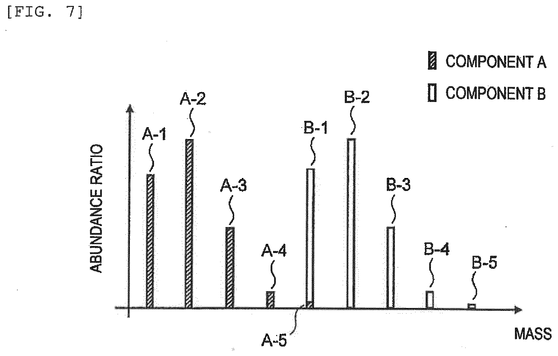

FIG. 7 is a conceptual diagram of the method for separating the ion intensity. Here, a component A and a component B (chlorinated paraffin) having different combinations of the number of carbon atoms x and the number of chlorine atoms y will be described as examples. FIG. 7 illustrates mass spectra calculated from masses and abundance ratios of the component A (component A-1 to component A-5) and the component B (component B-1 to component B-5), and five peaks appear respectively. The number following the letter of each component is the number of ³⁷Cl contained in each component. Therefore, when the number increases by 1, the mass increases by 2. In the example of FIG. 7, it is assumed that the masses of the component A-5 and the component B-1 are the same, that is, the peak positions overlap each other. In this case, the abundance ratios (vertical axis) of the non-overlapping peaks (component A-2) and the overlapping peaks (component A-5) can be calculated from the theoretical mass spectrum. Therefore, the ion intensity of the component A-5 of the mass spectrum obtained in step S3 can be calculated on the basis of the ion intensity of the component A-2 and the above ratio. The ion intensity of the component B-1 can also be acquired from a calculation result of the ion intensity of the component A-5. In this manner, even if there is a peak having the same mass between the component A and the component B, the ion intensities can be separated. Here, as described with reference to FIG. 3, the average value of the ion intensities in the predetermined width W of each peak can be used for the calculation of the ratio of the present step as the ion intensity of the peak.

The peak used in the processing of the present step (non-overlapping peaks between the homologs) is not limited, and a peak having the largest ion intensity may be used, or a plurality of peaks may be used. In addition, Step S8 is only required to be performed between step S3 and step S5, and an order of step S4 and step S8 is not limited.

### <Summary of Second Embodiment>

As described above, in the second embodiment, when there are components having the same mass between the homologs having different combinations of the number of carbon atoms x and the number of chlorine atoms y, the ion intensities of the components having the same mass are separated from the mass spectrum obtained by measuring the measurement target. Accordingly, even if homologs having the same mass (the peaks are at the same position) exist, since each homolog can be compared with the theoretical mass spectrum, an erroneous determination is prevented.

### [Third Embodiment]

In the third embodiment, a combination of the first embodiment and the second embodiment will be described.

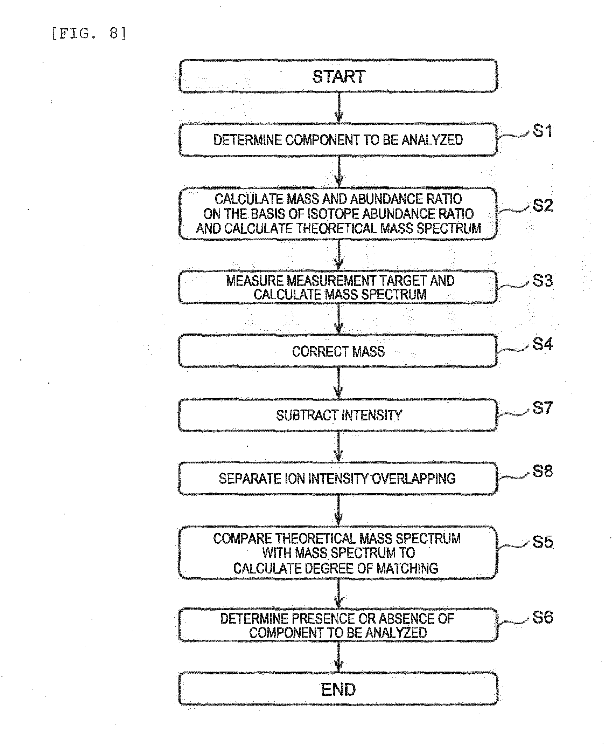

FIG. 8 is a flowchart of a mass spectrometry method according to the third embodiment. Contents of each processing of steps S1 to S8 are as described above. As illustrated in FIG. 8, step S7 and step S8 can be performed between step S4 and step S5. Step S7 is only required to be performed between step S3 and step S5, and may be performed before step S4, or may be performed after step S8.

### [Modification]

The disclosure is not limited to the above embodiments and configurations, and may include various modifications without departing from the subject-matter of the appended claims

### Reference Signs List

100: mass spectrometer
101: heating unit
102: ionization unit
103: detection Unit
104: control Unit

## Claims

1. A mass spectrometry method using a mass spectrometer (100), the mass spectrometry method comprising:
calculating, by a control unit (104) of the mass spectrometer (100), a theoretical mass spectrum by calculating, on the basis of a molecular formula of a component to be analyzed and an isotope abundance ratio of an element that is included in the component to be analyzed and for which a plurality of isotopes exist, a mass of the isotopes of the component to be analyzed and an abundance ratio of the component to be analyzed for each mass; and
ionizing, by a preprocessing unit of the mass spectrometer (100), a measurement target,
detecting, by a mass detection unit of the mass spectrometer (100), a mass of ionized ions and the number of ions in each mass;
calculating, by the control unit (104), a first mass spectrum on the basis of a detection result of the mass detection unit;
operating, by the control unit (104), the preprocessing unit and the mass detection unit in a state where the measurement target is absent;
calculating, by the control unit (104), a second mass spectrum when the measurement target is absent on the basis of the detection result of the mass detection unit; and
subtracting, by the control unit, the second mass spectrum from the first mass spectrum
so as to obtain a subtracted mass spectrum;
calculating, by the control unit (104), a degree of matching between the subtracted mass spectrum and the theoretical mass spectrum for only a mass in which a peak of the theoretical mass spectrum exists;
determining, by the control unit (104) and on the basis of the degree of matching, presence or absence of the component to be analyzed in the measurement target;
wherein
in the determination, the control unit compares the degree of matching with a first threshold and a second threshold larger than the first threshold, determines that the component to be analyzed is absent when the degree of matching is smaller than the first threshold, and determines that the component to be analyzed is present when the degree of matching is equal to or larger than the second threshold.

2. The mass spectrometry method according to claim 1, wherein
the component to be analyzed is an organic halogen compound, and
in calculating the theoretical mass spectrum, the control unit (104) calculates, on the basis of a molecular formula of the organic halogen compound and an isotope abundance ratio of halogen, masses of y + 1 types of isotopes of the organic halogen compound and an abundance ratio of the organic halogen compound for each mass, for each of combinations of the number of carbon atoms x and the number of halogen atoms y of the organic halogen compound.

3. The mass spectrometry method according to claim 1, further comprising:
separating, by the control unit (104), ion intensities of components having the same mass from the first mass spectrum when the components having the same mass are present between homologs of the component to be analyzed before calculating the degree of matching.

4. The mass spectrometry method according to claim 1, wherein
the control unit (104) calculates the degree of matching on the basis of an average ion intensity within a range of a predetermined width of the first mass spectrum.

5. The mass spectrometry method according to claim 1, wherein
the control unit (104) calculates the degree of matching by using a correlation coefficient of the number of ions of the mass in which the peak exists.

6. The mass spectrometry method according to claim 1, further comprising:
correcting, by the control unit (104), a mass of the first mass spectrum in accordance with an ionization reaction in the preprocessing unit.

7. The mass spectrometry method according to claim 1, wherein
the component to be analyzed is an organic chlorine compound.

8. The mass spectrometry method according to claim 1, wherein
the component to be analyzed is an organic bromine compound.

9. The mass spectrometry method according to claim 1, wherein
an ionization method of the preprocessing unit is atmospheric pressure chemical ionization method.

## Patentansprüche

1. Massenspektrometrieverfahren unter Verwendung eines Massenspektrometers (100), wobei das Massenspektrometrieverfahren Folgendes umfasst:
Berechnen durch eine Steuereinheit (104) des Massenspektrometers (100) eines theoretischen Massenspektrums durch Berechnen auf der Grundlage einer Summenformel einer Komponente, die analysiert werden soll, und eines Isotopenhäufigkeitsverhältnisses eines Elements, das in der Komponente, die analysiert werden soll, enthalten ist und für das mehrere Isotopen vorhanden sind, einer Masse der Isotopen der Komponente, die analysiert werden soll, und eines Häufigkeitsverhältnisses der Komponente, die analysiert werden soll, für jede Masse;
Ionisieren durch eine Vorverarbeitungseinheit des Massenspektrometers (100) eines Messungsziels;
Detektieren durch eine Massendetektionseinheit des Massenspektrometers (100) einer Masse ionisierter Ionen und der Anzahl von Ionen in jeder Masse;
Berechnen durch die Steuereinheit (104) eines ersten Massenspektrums auf der Grundlage eines Detektionsergebnisses der Massendetektionseinheit;
Betreiben durch die Steuereinheit (104) der Vorverarbeitungseinheit und der Massendetektionseinheit in einem Zustand, in dem das Messungsziel nicht vorhanden ist;
Berechnen durch die Steuereinheit (104) eines zweiten Massenspektrums, wenn das Messungsziel nicht vorhanden ist, auf der Grundlage des Detektionsergebnisses der Massendetektionseinheit und
Subtrahieren durch die Steuereinheit des zweiten Massenspektrums vom ersten Massenspektrum, um ein subtrahiertes Massenspektrum zu erhalten;
Berechnen durch die Steuereinheit (104) eines Grads der Übereinstimmung zwischen dem subtrahierten Massenspektrum und dem theoretischen Massenspektrum lediglich für eine Masse, in der ein Spitzenwert des theoretischen Massenspektrums vorhanden ist; und
Bestimmen durch die Steuereinheit (104) und auf der Grundlage des Grads der Übereinstimmung eines Vorliegens oder eines Fehlens der Komponente, die analysiert werden soll, im Messungsziel;
wobei die Steuereinheit in der Bestimmung den Grad der Übereinstimmung mit einem ersten Schwellenwert und einem zweiten Schwellenwert, der größer als der erste Schwellenwert ist, vergleicht und bestimmt, dass die Komponente, die analysiert werden soll, nicht vorhanden ist, wenn der Grad der Übereinstimmung kleiner als der erste Schwellenwert ist, und bestimmt, dass die Komponente, die analysiert werden soll, vorhanden ist, wenn der Grad der Übereinstimmung gleich oder größer als der zweite Schwellenwert ist.

2. Massenspektrometrieverfahren nach Anspruch 1, wobei
die Komponente, die analysiert werden soll, eine organische Halogenverbindung ist und
beim Berechnen des theoretischen Massenspektrums die Steuereinheit (104) auf der Grundlage einer Summenformel der organischen Halogenverbindung und eines Isotopenhäufigkeitsverhältnisses von Halogen Massen von y + 1 Typen von Isotopen der organischen Halogenverbindung und ein Häufigkeitsverhältnis der organischen Halogenverbindung für jede Masse für jede von Kombinationen der Anzahl von Kohlenstoffatomen x und der Anzahl von Halogenatomen y der organischen Halogenverbindung berechnet.

3. Massenspektrometrieverfahren nach Anspruch 1, das ferner Folgendes umfasst:
Trennen durch die Steuereinheit (104) von lonenintensitäten von Komponenten, die dieselbe Masse aufweisen, aus dem ersten Massenspektrum, wenn die Komponenten, die dieselbe Masse aufweisen, zwischen Homologen der Komponente, die analysiert werden soll, vorhanden sind, vor dem Berechnen des Grads der Übereinstimmung.

4. Massenspektrometrieverfahren nach Anspruch 1, wobei
die Steuereinheit (104) den Grad der Übereinstimmung auf der Grundlage einer durchschnittlichen Ionenintensität in einem Bereich einer vorgegebenen Breite des ersten Massenspektrums berechnet.

5. Massenspektrometrieverfahren nach Anspruch 1, wobei
die Steuereinheit (104) den Grad der Übereinstimmung unter Verwendung eines Korrelationskoeffizienten der Anzahl von Ionen der Masse, in der der Spitzenwert vorhanden ist, berechnet.

6. Massenspektrometrieverfahren nach Anspruch 1, das ferner Folgendes umfasst:
Korrigieren durch die Steuereinheit (104) einer Masse des ersten Massenspektrums gemäß einer Ionisierungsreaktion in der Vorverarbeitungseinheit.

7. Massenspektrometrieverfahren nach Anspruch 1, wobei
die Komponente, die analysiert werden soll, eine organische Chlorverbindung ist.

8. Massenspektrometrieverfahren nach Anspruch 1, wobei
die Komponente, die analysiert werden soll, eine organische Bromverbindung ist.

9. Massenspektrometrieverfahren nach Anspruch 1, wobei
ein Ionisierungsverfahren der Vorverarbeitungseinheit ein chemisches Ionisierungsverfahren bei Atmosphärendruck ist.

## Revendications

1. Procédé de spectrométrie de masse utilisant un spectromètre de masse (100), le procédé de spectrométrie de masse comprenant les étapes consistant à :
calculer, via une unité de commande (104) du spectromètre de masse (100), un spectre de masse théorique en calculant, sur la base d'une formule moléculaire d'un composant à analyser et d'un rapport d'abondance isotopique d'un élément qui est inclus dans le composant à analyser et pour lequel il existe une pluralité d'isotopes, une masse des isotopes du composant à analyser et un rapport d'abondance du composant à analyser pour chaque masse ; et
ioniser, via une unité de prétraitement du spectromètre de masse (100), une cible de mesure,
détecter, via une unité de détection de masse du spectromètre de masse (100), une masse d'ions ionisés et le nombre d'ions dans chaque masse ;
calculer, via l'unité de commande (104), un premier spectre de masse sur la base d'un résultat de détection de l'unité de détection de masse ;
actionner, via l'unité de commande (104), l'unité de prétraitement et l'unité de détection de masse dans un état dans lequel la cible de mesurage est absente ;
calculer, via l'unité de commande (104), un second spectre de masse quand la cible de mesurage est absente sur la base du résultat de détection de l'unité de détection de masse ; et
soustraire, via l'unité de commande (104), le second spectre de masse du premier spectre de masse de manière à obtenir un spectre de masse soustrait ;
calculer, via l'unité de commande (104), un degré de correspondance entre le spectre de masse soustrait et le spectre de masse théorique pour une seule masse dans laquelle un pic du spectre de masse théorique existe ;
déterminer, via l'unité de commande (104) et sur la base du degré de correspondance, une présence ou une absence du composant à analyser dans la cible de mesurage ;
dans lequel, dans la détermination, l'unité de commande compare le degré de correspondance à un premier seuil et à un second seuil supérieur au premier seuil, détermine que le composant à analyser est absent quand le degré de correspondance est inférieur au premier seuil, et détermine que le composant à analyser est présent quand le degré de correspondance est égal ou supérieur au second seuil.

2. Procédé de spectrométrie de masse selon la revendication 1, dans lequel le composant à analyser est un composé halogène organique, et
dans le calcul du spectre de masse théorique, l'unité de commande (104) calcule, sur la base d'une formule moléculaire du composé halogène organique et d'un rapport d'abondance isotopique d'halogène, des masses de y + 1 types d'isotopes du composé halogène organique et un rapport d'abondance du composé halogène organique pour chaque masse, pour chacune de combinaisons du nombre d'atomes de carbone x et du nombre d'atomes d'halogène y du composé halogène organique.

3. Procédé de spectrométrie de masse selon la revendication 1, comprenant en outre l'étape consistant à :
séparer, via l'unité de commande (104), des intensités ioniques de composants ayant la même masse du premier spectre de masse quand les composants ayant la même masse sont présents entre des homologues du composant à analyser avant de calculer le degré de correspondance.

4. Procédé de spectrométrie de masse selon la revendication 1, dans lequel l'unité de commande (104) calcule le degré de correspondance sur la base d'une intensité ionique moyenne à l'intérieur d'une plage d'une largeur prédéterminée du premier spectre de masse.

5. Procédé de spectrométrie de masse selon la revendication 1, dans lequel l'unité de commande (104) calcule le degré de correspondance en utilisant un coefficient de corrélation du nombre d'ions de la masse dans laquelle le pic existe.

6. Procédé de spectrométrie de masse selon la revendication 1, comprenant en outre l'étape consistant à :
corriger, via l'unité de commande (104), une masse du premier spectre de masse en accord avec une réaction d'ionisation dans l'unité de prétraitement.

7. Procédé de spectrométrie de masse selon la revendication 1, dans lequel le composant à analyser est un composé de chlorine organique.

8. Procédé de spectrométrie de masse selon la revendication 1, dans lequel le composant à analyser est un composé de brome organique.

9. Procédé de spectrométrie de masse selon la revendication 1, dans lequel une technique d'ionisation de l'unité de prétraitement est une technique d'ionisation chimique à pression atmosphérique.
